(19) **Europäisches Patentamt / European Patent Office / Office européen des brevets**

(11) **EP 4 039 170 A1**

(12) # EUROPEAN PATENT APPLICATION
published in accordance with Art. 153(4) EPC

(43) Date of publication:
**10.08.2022 Bulletin 2022/32**

(21) Application number: **20870601.0**

(22) Date of filing: **24.08.2020**

(51) International Patent Classification (IPC):
**A61B 3/08** (2006.01)    **G02B 30/00** (2020.01)
**G02C 7/02** (2006.01)

(52) Cooperative Patent Classification (CPC):
**A61B 3/08; G02B 30/00; G02C 7/02**

(86) International application number:
**PCT/JP2020/031783**

(87) International publication number:
**WO 2021/065247 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2019  JP 2019180309**

(71) Applicant: **Hoya Lens Thailand Ltd.**
**Pathumthani 12130 (TH)**

(72) Inventors:
• **ITO, Ayumu**
 **Tokyo 160-8347 (JP)**
• **SONEHARA, Toshiaki**
 **Tokyo 160-8347 (JP)**
• **ISHIHARA, Nagisa**
 **Tokyo 160-8347 (JP)**
• **YAMAGUCHI, Eiichiro**
 **Tokyo 160-8347 (JP)**

(74) Representative: **Betten & Resch**
**Patent- und Rechtsanwälte PartGmbB**
**Maximiliansplatz 14**
**80333 München (DE)**

(54) **BINOCULAR VISION FUNCTION MEASUREMENT METHOD, BINOCULAR VISION FUNCTION MEASUREMENT PROGRAM, SPECTACLE LENS DESIGNING METHOD, SPECTACLE LENS MANUFACTURING METHOD, AND BINOCULAR VISION FUNCTION MEASUREMENT SYSTEM**

(57)   Provided is a binocular visual function measurement method including a visual target presentation step of presenting a right eye image to be viewed by the right eye of a measurement subject and a left eye image to be viewed by the left eye of the measurement subject to the measurement subject on a single portable display screen; a presentation control step of changing positions where the right eye image and the left eye image are presented, relative to each other; a timing detection step of detecting a timing at which the measurement subject is unable to fuse the right eye image and the left eye image when the presentation positions are changed; and a parameter value calculation step of calculating a predetermined parameter value regarding a binocular visual function of the measurement subject based on a relationship between the relative positions of the right eye image and the left eye image when the timing is detected.

**FIG. 3**

113a Presentation control unit    113c Parameter value calculation unit
113b Timing detection unit        113d Tracking ability determination unit

EP 4 039 170 A1

**Description**

Technical Field

The present invention relates to a binocular visual function measurement method, a binocular visual function measurement program, an eyeglass lens designing method, an eyeglass lens manufacturing method, and a binocular visual function measurement system.

Background Art

Eyeglass wearers may be subjected to binocular visual function examinations to measure convergence and divergence ranges, for example. There are individual differences in convergence and divergence ranges, and it is very important to measure the convergence and divergence ranges in order to understand the functions of the eyes in near vision in designing of eyeglass lenses.

With regard to the binocular visual functions represented by the convergence and divergence ranges and the like, Patent Document 1 discloses that the binocular visual function is measured by presenting left and right parallax images using a stationary three-dimensional compatible video monitor, moving the positions where they are presented, relative to each other, and detecting the timing at which images cannot be fused, for example.

Citation List

Patent Documents

Patent Document 1: JP-2012-95693A

Summary of Invention

Technical Problem

In the binocular visual function measurement method disclosed in Patent Document 1, the binocular visual function is measured using a stationary three-dimensional compatible video monitor. Thus, the position of the head of a measurement subject is not fixed with respect to left and right parallax images, and thus there is a risk that an error will occur in the median plane depending on the orientation of the head during measurement. Furthermore, by placing the stationary three-dimensional compatible video monitor in a real environment, in addition to parallax information to be displayed, the measurement subject simultaneously acquires information (real space information) that gives a sense of depth and perspective from the outside world, which may result in the convergence and divergence that occur together with normal accommodation. Furthermore, because the stationary three-dimensional compatible video monitor is used, the size of the required system configuration is increased, and thus it cannot be said that the binocular visual function can be easily measured.

The present invention aims to provide a technique by which the binocular visual function of a measurement subject can be very easily measured with high accuracy.

Solution to Problem

The present invention was made to achieve the above-described aim.

A first aspect of the present invention is directed to a binocular visual function measurement method, the method including:

a visual target presentation step of presenting a right eye image to be viewed by the right eye of a measurement subject and a left eye image to be viewed by the left eye of the measurement subject to the measurement subject on a single portable display screen;
a presentation control step of changing positions where the right eye image and the left eye image are presented, relative to each other;
a timing detection step of detecting a timing at which the measurement subject is unable to fuse the right eye image and the left eye image when the presentation positions are changed; and
a parameter value calculation step of calculating a predetermined parameter value regarding a binocular visual function of the measurement subject based on a relationship between the relative positions of the right eye image and the left eye image when the timing is detected.

A second aspect of the present invention is directed to the binocular visual function measurement method according to the first aspect,
in which the right eye image and the left eye image are presented using a display screen of a mobile information terminal as the portable display screen.

A third aspect of the present invention is directed to the binocular visual function measurement method according to the first or second aspect,
in which the predetermined parameter value is a value for specifying a convergence range of the measurement subject.

A fourth aspect of the present invention is directed to the binocular visual function measurement method according to any one of the first to third aspects, the method including
a tracking ability determination step of determining a level of the ability of an eye of the measurement subject to track a change in positions of the presented images by changing the speed of a change in the relative positions of the right eye image and the left eye image and acquiring a plurality of the predetermined parameter values.

A fifth aspect of the present invention is directed to the binocular visual function measurement method according to any one of the first to fourth aspects,
in which the right eye image and the left eye image are constituted by figures having the same shape and the same size.

A sixth aspect of the present invention is directed to the binocular visual function measurement method according to any one of the first to fifth aspects, the method including
a visual range setting step of setting a visual range of the measurement subject with respect to the right eye image and the left eye image.

A seventh aspect of the present invention is directed to a binocular visual function measurement program for causing a computer to execute the binocular visual function measurement method according to any one of the first to sixth aspects.

An eighth aspect of the present invention is directed to an eyeglass lens designing method, the method including:

a step of measuring the binocular visual function of the measurement subject using the binocular visual function measurement method according to any one of the first to sixth aspects; and
a step of determining an optical design value of the eyeglass lens based on a result of the measurement of the binocular visual function.

A nineth aspect of the present invention is directed to an eyeglass lens manufacturing method, the method including:

a step of designing an eyeglass lens using the eyeglass lens designing method according to the eighth aspect; and
a step of manufacturing the eyeglass lens according to a result of designing the eyeglass lens.

A tenth aspect of the present invention is directed to a binocular visual function measurement system, the system including:

a visual target presentation unit configured to present a right eye image to be viewed by the right eye of a measurement subject and a left eye image to be viewed by the left eye of the measurement subject to the measurement subject on a single portable display screen;
a presentation control unit configured to change positions where the right eye image and the left eye image are presented, relative to each other;
a timing detection unit configured to detect a timing at which the measurement subject is unable to fuse the right eye image and the left eye image when the presentation positions are changed; and
a parameter value calculation unit configured to calculate a predetermined parameter value regarding a binocular visual function of the measurement subject based on a relationship between the relative positions of the right eye image and the left eye image when the timing is detected.

An eleventh aspect of the present invention is directed to the binocular visual function measurement system according to the tenth aspect,
in which the visual target presentation unit is configured to present the right eye image and the left eye image using a display screen of a mobile information terminal as the portable display screen.

Advantageous Effects of Invention

According to the present invention, the binocular visual function of a measurement subject can be very easily measured with high accuracy.

Brief Description of Drawings

FIG. 1 is a block diagram showing a configuration of an eyeglass lens manufacturing system for realizing an eyeglass lens manufacturing method according to an embodiment of the present invention.

FIG. 2 is a diagram showing an overview of the binocular visual function measurement system according to the embodiment of the present invention.

FIG. 3 is a block diagram showing a configuration of the binocular visual function measurement system according to the embodiment of the present invention.

FIG. 4 is a diagram showing a flowchart of processing executed in a convergence range measurement mode by a binocular visual function measurement program according to the embodiment of the present invention.

FIG. 5 shows transition diagrams of images displayed on a display screen during execution of the convergence range measurement mode according to the embodiment of the present invention.

FIG. 6 is a diagram showing a flowchart of a variation of processing executed in the convergence range measurement mode by the binocular visual function measurement program according to the embodiment of the present invention.

FIG. 7 is a diagram showing a flowchart of processing executed in a left-right eye vertical divergence allowable value measurement mode by the binocular visual function measurement program according to the embodiment of the present invention.

FIG. 8 shows transition diagrams of images displayed on a display screen during execution of the left-right eye vertical divergence allowable value measurement mode according to the embodiment of the present invention.

FIG. 9 is a diagram showing a flowchart of processing executed in a first unequal magnification allowable value measurement mode by the binocular visual function measurement program according to the embodiment of the present invention.

FIG. 10 shows transition diagrams of images displayed on the display screen during execution of the first unequal magnification allowable value measurement mode according to the embodiment of the present invention.

FIG. 11 is a diagram showing a flowchart of processing executed in a second unequal magnification allowable value measurement mode by the binocular visual function measurement program according to the embodiment of the present invention.

FIG. 12 shows transition diagrams of transition diagrams of images displayed on the display screen during execution of the second unequal magnification allowable value measurement mode according to the embodiment of the present invention.

FIG. 13 shows a diagram for describing Listing's law.

FIG. 14 is a diagram illustrating the line-of-sight direction of the left and right eyes in the case of binocular vision.

FIG. 15 is a diagram showing a flowchart of processing executed in a left-right eye rotation parallax allowable value measurement mode by the binocular visual function measurement program according to the embodiment of the present invention.

FIG. 16 shows transition diagrams of images displayed on the display screen during execution of the left-right eye rotation parallax allowable value measurement mode according to an embodiment of the present invention.

FIG. 17 shows a transition diagram of images in a first composite measurement mode.

FIG. 18 shows a transition diagram of images in a second composite measurement mode.

FIG. 19 shows a transition diagram of images in a third composite measurement mode.

FIG. 20 shows a transition diagram of images in a measurement mode considering a lateral view.

Description of Embodiments

Hereinafter, an embodiment of the present invention will be described with reference to the drawings.

<Overview of Embodiment

First, an overview of this embodiment will be described.

In the present embodiment, a portable mobile information terminal is used, and a pair of images with parallax are displayed on a single display screen (also referred to as a "portable display screen" hereinafter) of the mobile information terminal, and are respectively presented to the left and right eyes of a measurement subject. The binocular visual function of the measurement subject is measured by determining whether the images are fused (identified) when the given parallax is changed. The binocular visual function is measured by calculating a predetermined parameter value for the binocular visual function.

Examples of the predetermined parameter value include values for specifying a convergence range of the measurement subject. A "convergence range" herein refers to the difference in angle between the convergence limit and the divergence limit. Note that the angle difference may be expressed by the refractive power of a prism. In the following description, a case where the convergence range is measured will mainly be described as an example. The predetermined parameter value is not limited to the values in the convergence range, and may be another parameter value such as the left-right eye vertical divergence allowable value, the first unequal magnification allowable value, the second unequal magnification allowable value, or the left-right eye rotation parallax allowable value, which will be described later.

In the present embodiment, when the binocular visual function is measured, the parallax images are presented to the measurement subject by positioning the portable display screen of the mobile information terminal in front of the eyes of the measurement subject. Therefore, the parallax images can be presented very easily.

Furthermore, when the portable display screen is positioned in front of the eyes of the measurement subject, by screening out the surrounding region of the portable display screen, the parallax images can be presented to the measurement subject in a space where real space information is blocked out. That is, the parallax images are presented in front of the eyes of the measurement subject in a state where the outside world is screened out, and thus, the measurement subject does not acquire information (real space information) that gives a sense of depth and perspective from the outside world, in addition to the presented parallax images.

Furthermore, by keeping the portable display screen in front of the eyes of the measurement subject, the parallax images can be can be accurately positioned regardless of the direction of the face of the measurement subject.

Also, if the visual range to the parallax images (the physical distance between the images and the subject who sees the images) is also kept constant, it is possible to measure the binocular visual function while keeping the accommodation function (focusing function) of the eyes of the measurement subject constant.

Therefore, according to the present embodiment, by positioning the portable display screen in front of the eyes of the measurement subject, it is possible to perform composite measurements in the same measurement environment without taking the posture and the position of the measurement subject into consideration. Also, by presenting only a pair of parallax images using the portable display screen of the mobile information terminal, it is possible to measure the capability relating to the convergence range without relying on a sense of depth, and to reduce the influence of accommodative convergence. "Accommodative convergence" here refers to convergence (convergence and divergence movement) that occurs simultaneously with accommodation that occurs according to the visual range.

That is, according to the present embodiment, it is possible to very easily measure the convergence range while suppressing three-dimensional perception by the measurement subject. If the convergence range of the measurement subject can be very easily measured with high accuracy in this manner, an eyeglass lens suitable for the measurement subject can be provided by using the results of measurements as one of the parameters for designing the eyeglass lens.

For example, a measurement subject who has been found to have weak motor fusion based on the results of measurements is likely to complain of diplopia in which images are unlikely to fuse under strong binocular separation. Therefore, it is possible to provide an eyeglass lens with a lens design that compensates for weak motor fusion by inserting a prism to the extent where motor fusion is achieved. Furthermore, with regard to the measurement subject who was found to have strong motor fusion based on the results of measurement, the Panum's fusional area may be wide, and thus it is possible to provide an eyeglass lens provided with a lens design that can reduce inset without affecting fusion of images and reduce the maximum aberration on the nasal side, for example. Note that "motor fusion" refers to fusion with eyeball movements performed to maintain single vision.

<Example of system configuration>

Next, a specific content of the present embodiment will be described.

(Configuration of eyeglass lens manufacturing system)

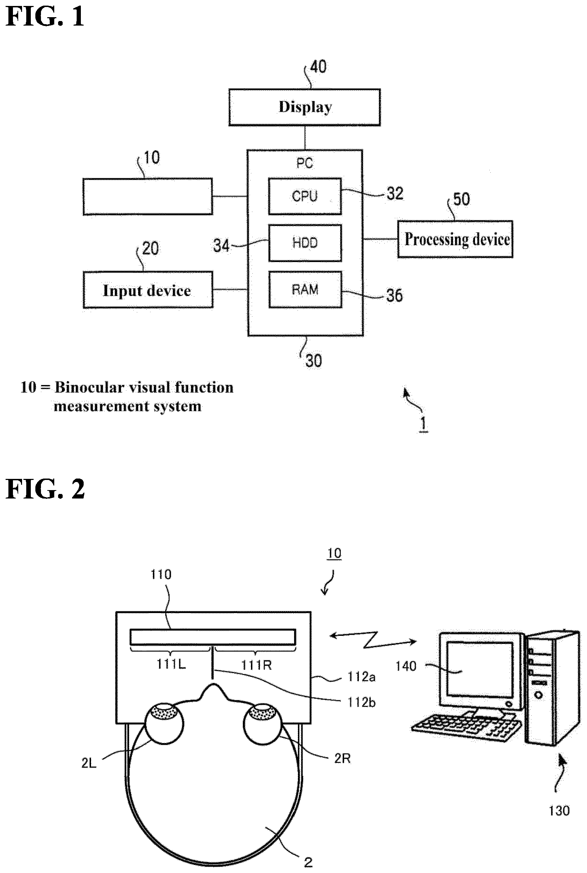

FIG. 1 is a block diagram showing a configuration of an eyeglass lens manufacturing system 1 for realizing an eyeglass lens manufacturing method according to the present embodiment. The eyeglass lens manufacturing system 1 is installed in an eyeglass lens manufacturing factory, for example, and as shown in FIG. 1, the eyeglass lens manufacturing system 1 includes a binocular visual function measurement system 10, an input device 20 (a keyboard, a mouse, a game controller, and the like), a PC (Personal Computer) 30, a display 40, and a processing device 50. The PC 30 receives data regarding measurement of the binocular visual function of a measurement subject measured using the binocular visual function measurement system 10, and data regarding the specifications of an eyeglass lens that was input to the input device 20. Specification data includes the optical properties and product type of eyeglass lens, for example. Vertex refractive power (spherical refractive power, cylindrical power, cylindrical axis direction, prismatic refractive power, and prism base direction) and the like are considered as being the optical properties of an eyeglass lens, for example. The binocular visual function measurement system 10 and the input device 20 may be installed in an optician's store away from an eyeglass lens manufacturing plant. In this case, data measured by the binocular visual function measurement system 10 and the specification data input to the input device 20 are transmitted to the PC 30 via a computer network.

The PC 30 includes a CPU (Central Processing Unit) 32, an HDD (Hard Disk Drive) 34, and a RAM (Random Access Memory) 36. A processing control program for controlling the processing device 50 is installed in the HDD 34. The CPU 32 loads the processing control program onto the RAM 36, and starts the program. When the processing control program is started, a GUI (Graphical User Interface) for issuing an instruction to design and manufacture an eyeglass lens is displayed on a display screen of the display 40. The processing control program selects a semi-finished lens based on specification data and measurement data, performs surface shape optimization calculation, and determines optical design values.

An operator sets the selected semi-finished lens in the processing device 50, operates the GUI, and inputs an instruction to start processing. The processing control program reads the determined optical design values and controls driving of the processing device 50. The processing device 50 grinds the surface of the semi-finished lens according to the execution of the processing control program so as to manufacture an eyeglass lens. Note that a specific method for designing an eyeglass lens using measurement data regarding the binocular visual function is described in a pamphlet in WO 2010/090144 filed by the applicant, for example.

(Configuration of binocular visual function measurement system)

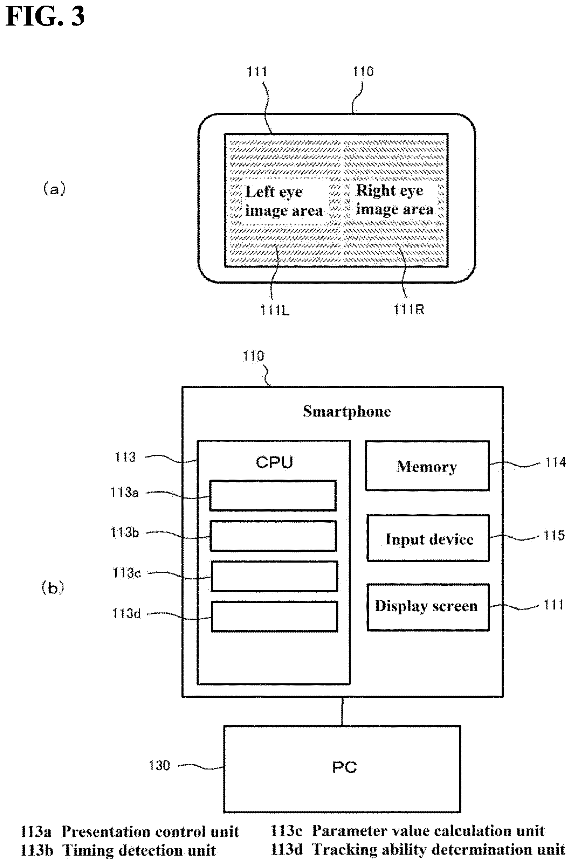

FIG. 2 is a diagram showing an overview of the binocular visual function measurement system 10. FIG. 3 is a block diagram showing a configuration of the binocular visual function measurement system 10. In the binocular visual function measurement method using the binocular visual function measurement system 10, multiple types of binocular visual functions of a measurement subject 2 can be measured in order to obtain design data (or evaluation data) regarding an eyeglass lens that cannot be obtained using a prescription obtained focusing on only one eye.

As shown in FIGS. 2 and 3, the binocular visual function measurement system 10 includes a smartphone 110, which is a mobile information terminal. The mobile information terminal is a small information electronic device that the user can carry, and is configured including at least a display screen that displays information. The smartphone 110 is one type of mobile information terminal, and is configured to function as a small computer device, in addition to functioning as a mobile phone. Note that, although a case where the mobile information terminal is the smartphone 110 is described here as an example, there is no limitation to this, and other mobile information terminals such as a tablet terminal and a PDA (Personal Digital Assistant) may also be used, for example.

The smartphone 110 includes a display screen (portable display screen) 111 constituted by a single LCD (Liquid Crystal Display) panel, an organic EL (electroluminescence) panel, or the like on one side thereof. An area of the display screen 111 is divided into a right eye image area 111R and a left eye image area 111L. Also, as will be described later in detail, a configuration is adopted in which a right eye image to be viewed by a right eye 2R of the measurement subject 2 is displayed in the right eye image area 111R and a left eye image to be viewed by a left eye 2L of the measurement subject 2 is displayed in the left eye image area 111L.

Also, the smartphone 110 is supported by a support housing portion 112a such that the display screen 111 is positioned in front of the eyes of the measurement subject 2. That is, when the support housing portion 112a is worn on the head portion of the measurement subject 2, the display screen 111 of the smartphone 110 supported by the support housing portion 112a is positioned in front of the eyes of the measurement subject 2. In the smartphone 110 supported in this manner, the display screen 111 is disposed in the closed space formed by the support housing portion 112a, and thus, images are displayed to the measurement subject 2 in a space where information (real space information) that gives a sense of depth and perspective is blocked out from the outside world.

It is preferable that a partition wall 112b is provided in the closed space formed by the support housing portion 112a so as to be located between the right eye image area 111R and the left eye image area 111L of the display screen 111. This makes it possible to inhibit light from the right eye image area 111R of the display screen 111 from reaching the left eye 2L of the measurement subject 2, and to inhibit light from the left eye image area 111L from reaching the right eye 2R of the measurement subject 2.

That is, as a result of the smartphone 110 being supported by the support housing portion 112a worn on the head portion of the measurement subject 2, the smartphone 110 functions as a "visual target presentation unit" that presents the right eye image and the left eye image to the measurement subject 2 using a single display screen 111 in a space where real space information is blocked out. In other words, the visual target presentation unit is constituted using the smartphone 110 located in front of the eyes of the measurement subject 2.

When such a smartphone 110 is positioned in front of the eyes of the measurement subject 2, only the right eye image is visible to the right eye 2R of the measurement subject 2, and only the left eye image is visible to the left eye 2L of the measurement subject 2. As a result, the measurement subject 2 can fuse the parallax images of the right eye image area 111R and the left eye image area 111L even if images are formed at non-corresponding points on the retina within the Panum's fusional area.

Although it is conceivable to form the support housing portion 112a that supports the smartphone 110 by molding a resin material, for example, there is no limitation to this, and the support housing portion 112a may be formed of another material (e.g., a paper material or a metal material). The same also applies to the partition wall 112b.

Note that the support housing portion 112a that supports the smartphone 110 may have the function of being able to vary the settings of the visual range of the measurement subject 2 with respect to the right eye image and the left eye image. Specifically, in order to achieve a variable visual range, a configuration may be adopted in which a plurality of spacers (frame members) are prepared, and the visual range of the support housing portion 112a can be varied by selectively installing any of the plurality of spacers, for example. Furthermore, a lens having a given refractive power may be installed between the left eye 2L and the left eye image area 111L and/or between the right eye 2R and the right eye image area 111R. The number of lenses may be one, or a plurality of lenses may be used in combination to achieve a desired refractive power.

Furthermore, the smartphone 110 includes a CPU 113, a memory 114, and an input device 115 in addition to the display screen 111, and the smartphone 110 is configured to function as a small computer device. An example of the input device 115 is a touch panel disposed to overlap the display screen 111, and a wireless keyboard that uses short-range wireless communication is preferably provided in addition to a touch panel, in order to improve the operability for an operator, the measurement subject 2, and the like.

A binocular visual function measurement program, which is a dedicated application for measuring a binocular visual function, is downloaded (installed) in the memory 114. The CPU 113 reads the binocular visual function measurement program from the memory 114, and starts the binocular visual function measurement program. When the binocular visual function measurement program is started, the CPU 113 functions as a presentation control unit 113a, a timing detection unit 113b, and a parameter value calculation unit 113c.

The presentation control unit 113a controls image display operations in the right eye image area 111R and the left eye image area 111L of the display screen 111. Specifically, the presentation control unit 113a instructs the right eye image area 111R to present a right eye image and instructs the left eye image area 111L to present a left eye image, and the presentation control unit 113a changes positions where the right eye image and the left eye image are presented, relative to each other. A specific mode in which the presentation positions are changed relative to each other will be described later in detail.

The right eye image and the left eye image presented by the presentation control unit 113a function as parallax images, and thus it is presumed that they are formed based on figures having the same shape and the same size. Note that specific examples of the figures constituting parallax images will be described later in detail.

When the presentation positions of the right eye image and the left eye image are changed relative to each other, the timing detection unit 113b detects the timing at which the measurement subject 2 cannot fuse the right eye image and the left eye image. Such timing may be detected based on the content of an operation made by the measurement subject 2 on the input device 115.

When the parameter value calculation unit 113c detects the timing at which the timing detection unit 113b detects that images cannot be fused, the parameter value calculation unit 113c calculates a predetermined parameter value for the binocular visual function of the measurement subject 2 based on the relationship between the relative positions of the right eye image and the left eye image. Specific examples of the predetermined parameter value will be described later in detail.

In addition to these functions, the CPU 113 may function as a tracking ability determination unit 113d in response to the start of the binocular visual function measurement program.

The tracking ability determination unit 113d determines the level of the tracking ability of an eye of the measurement subject 2 with respect to a change in the position of a presented image. Specifically, by performing multiple measurements with different speeds of change in the relative positions of the right eye image and the left eye image when the binocular visual function of the measurement subject 2 is measured, it is determined which level the tracking ability of the eye of the measurement subject 2 corresponds to out of multiple preset levels.

Even if the relative positions of the right eye image and the left eye image change at a high speed, when the calculated predetermined parameter value does not largely change from that at a low speed, it is conceivable that the level of the tracking ability of the eye of the measurement subject 2 is high. In general, it is conceivable that it is easier to handle parallax that changes at a low speed than at a high speed, and thus, it is also possible to judge the level of the tracking ability of the eye of the measurement subject 2 using the speed dependence based on the parameter value acquired in a given slow change.

A PC 130 is connected to the smartphone 110 configured as described above via a wired or wireless communication line. A display 140 is connected to the PC 130.

Note that, in the system configuration described above, if the smartphone 110 and the PC 130 can constantly communicate with each other, the functions of the presentation control unit 113a, the timing detection unit 113b, the parameter value calculation unit 113c, and the tracking ability determination unit 113d that are realized by the smartphone 110, and the function of the input device 115 may also be realized by the PC 130.

Furthermore, in the system configuration described above, if all of the constituent elements of the eyeglass lens manufacturing system 1 are installed at the same location, the PC 30 shown in FIG. 1 and the PC 130 shown in FIG. 2 or 3 may be a single PC. Also, the input device 20 shown in FIG. 1 and the input device 115 shown in FIG. 2 or 3 may be a single input device. Furthermore, the display 40 shown in FIG. 1 and the display 140 shown in FIG. 2 or 3 may be a single display.

<Procedure for measuring binocular visual function>

Next, specific content of a procedure for measuring a binocular visual function using the binocular visual function measurement system 10 configured as described above, that is, the binocular visual function measurement method according to the present embodiment, will be described.

If a binocular visual function is to be measured, the binocular visual function measurement program is started in the smartphone 110, and the GUI for giving various instructions for measuring the binocular visual function is displayed on the display screen 111. Also, when the operator operates the GUI, the binocular visual function measurement program generates measurement data in accordance with the GUI operation. Furthermore, when the smartphone 110 is supported by the support housing portion 112a and is positioned in front of the eyes of the measurement subject 2, the smartphone 110 processes measurement data, generates the right eye image and the left eye image for measuring the binocular visual function, and displays the images in the right eye image area 111R or the left eye image area 111L of the display screen 111. This starts the measurement of the binocular visual function.

The binocular visual function measurement program supports various measurement items relating to the binocular visual function, and outputs the parameter values for the various measurement items as the results of the measurement. Examples of the supported parameter values include the convergence range, the left-right eye vertical divergence allowable value, the first unequal magnification allowable value, the second unequal magnification allowable value, and the left-right eye rotation parallax allowable value. When the operator measures the binocular visual function, the operator selects any one of the measurement items on the GUI.

Furthermore, the operator inputs the age, visual range, and the like as measurement conditions. The input measurement conditions are stored in the memory 114. Note that, with regard to the visual range, the visual range may be changed as needed if the smartphone 110 or the support housing portion 112a has the function of being able to vary the visual range settings.

The following describes processing executed by the binocular visual function measurement program when each of the above-listed measurement items is selected.

(If "convergence range" is selected)

The binocular visual function measurement program transitions to the convergence range measurement mode in which the convergence range of the measurement subject 2 is measured. The "convergence range" herein refers to convergence without accommodation. Here, as indicated by a known Donders diagram, the convergence (or divergence) of eyeballs and accommodation originally occur together. Therefore, it is not easy to measure convergence separately from accommodation. Note that the Donders diagram is described in a document "written by Shinobu Ishihara and revised by Shinichi Shikano, "Little Pupil Science" 17th revised version, Kanehara & Co. Ltd., (1925) p50", a document "written by Toyohiko Hatada, "Depth Information and Characteristics of Vision", Visual Information Research Group, April 23, 1974, p12", and WO 2010/090144 pamphlet filed by the applicant, and the like. In the Donders diagram, a straight line passing through the origin and having a slope 1 (angle of 45 degrees) is the Donders line. The Donders line represents cooperation between convergence and accommodation when a measurement subject who does not have strabismus or heterophoria is looking at an object with naked eyes. A Donders curve indicating the limit of the convergence (or the divergence) is plotted on the left and right sides of the Donders line. Values from one point on the Donders line to the right Donders curve (the side where the convergence angle is large) are classified into negative relative convergence, and values from one point thereon to the left Donders curve (the side where the convergence angle is small) are classified into positive relative convergence.

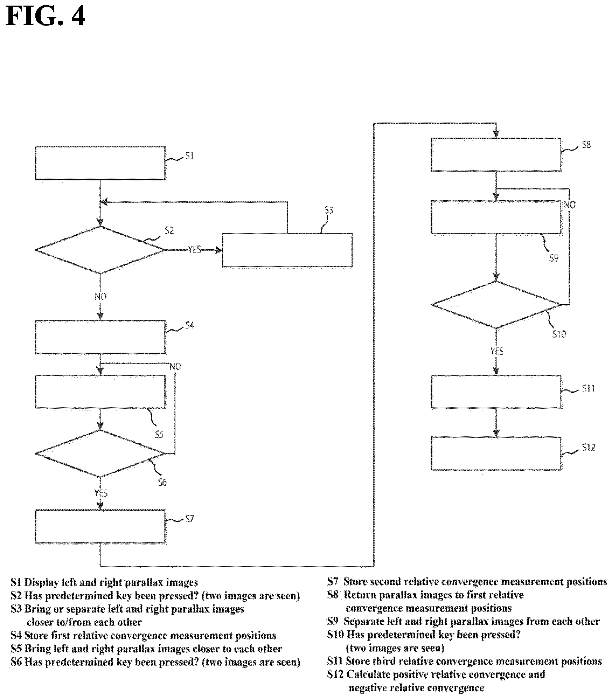

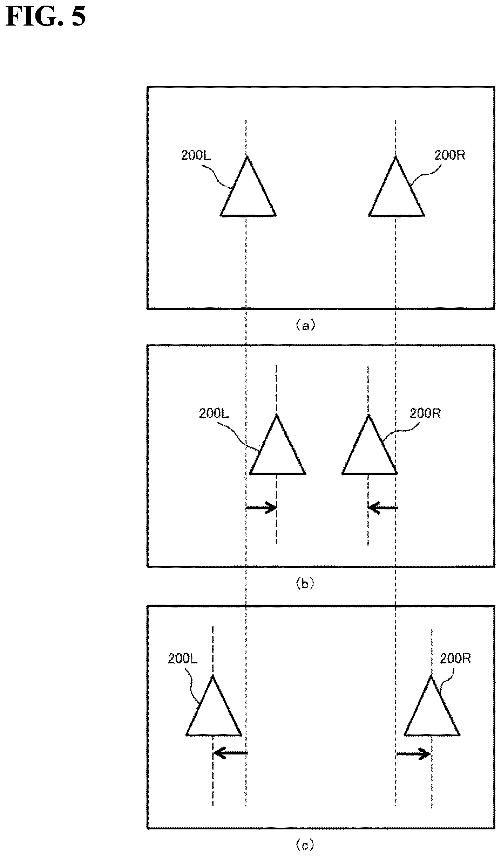

FIG. 4 is a diagram showing a flowchart of processing executed by the binocular visual function measurement program in a convergence range measurement mode. FIG. 5 shows transition diagrams of images displayed on the display screen during execution of the convergence range measurement mode. A processing step is abbreviated as "S" in the following description of this specification and drawings.

As shown in FIG. 5(a), when transitioning to the convergence range measurement mode, the left eye image 200L and the right eye image 200R are displayed in the left eye image area 111L and the right eye image area 111R of the display screen 111 (S1 in FIG. 4). The left eye image 200L and the right eye image 200R are identical images with the same size, color, shape, and the like. It is desirable that the left eye image 200L and the right eye image 200R have a simple geometrical shape such that the measurement subject 2 can focus on measurement. Although the drawings show a case where the left eye image 200L and the right eye image 200R are triangular figures, there is no limitation to this, and these images may be figures of a straight line (line segment) extending in the same direction over the same length, figures with other geometrical shapes, or the like, for example.

Furthermore, the measurement subject 2 is instructed to perform a predetermined operation such as pressing an operation key of the input device 115 when the measurement subject 2 sees two images. An instruction is displayed in at least one of the right eye image area 111R and the left eye image area 111L, for example. Also, an operator may give an instruction directly to the measurement subject 2. The same instruction is issued even when measurement items other than the convergence range are measured.

As shown in FIG. 5(a), in the processing of S2 shown in FIG. 4, the left eye image 200L and the right eye image 200R are respectively displayed in the right eye image area 111R and the left eye image area 111L, and it is checked whether the measurement subject 2 sees two images. If the measurement subject 2 sees two images, in the processing of S3 shown in FIG. 4, the operator separates or brings the right eye image area 111R and the left eye image area 111L closer to positions where two images cannot be seen, and repeats this operation until two images cannot be seen.

Specifically, if the operator separates these areas, for example, as shown in FIG. 5(c), the left eye image 200L and the right eye image 200R move in the horizontal direction (the directions indicated by the arrows in FIG. 5(c)) of the right eye image area 111R and the left eye image area 111L, and separate from each other. The movements of images that separate from each other are rendered in a continuously changing manner or in an incrementally changing manner. The left eye image 200L and the right eye image 200R continue to be separated from each other until the predetermined operation key of the input device 115 is pressed (NO in S2 in FIG. 4). When the predetermined operation key is pressed by the measurement subject 2, the amounts of positional shift of the left eye image 200L and the right eye image 200R at this time (hereinafter referred to as "first relative convergence measurement positions" for convenience of description) are stored in the memory 114 (S4 in FIG. 4). Note that the positions of the left eye image 200L and the right eye image 200R need only relatively separate from each other. Therefore, the position of either the left eye image 200L or the right eye image 200R may be fixed during measurement. The same applies to a left-right eye vertical divergence allowable value measurement mode, which will be described later.

The period of time during which separating images are presented is set as appropriate according to the tracking ability of a measurement subject.

In the processing of S5 in FIG. 4, the left eye image 200L and the right eye image 200R move from the first relative convergence measurement positions in the horizontal direction (the directions indicated by the arrows in FIG. 5(b)) of the right eye image area 111R and the left eye image area 111L, and approach each other. The left eye image 200L and the right eye image 200R continue to approach each other until the predetermined operation key of the input device 115 is pressed (YES in S6 in FIG. 4). When the predetermined operation key is pressed by the measurement subject 2, the amounts of positional shift of the left eye image 200L and the right eye image 200R at this time (hereinafter referred to as "second relative convergence measurement positions" for convenience of description) are stored in the memory 114 (S7 in FIG. 4).

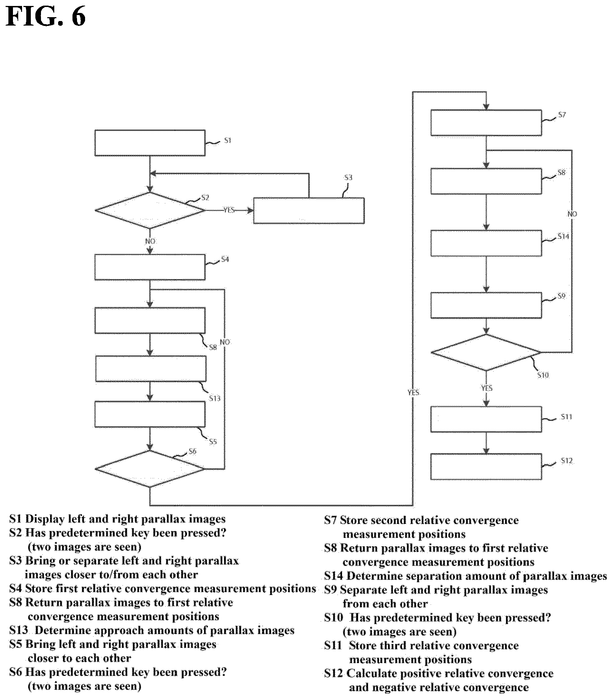

Then, in the processing of S8 in FIG. 4, the left eye image 200L and the right eye image 200R are returned from the second relative convergence measurement positions to the first relative convergence measurement positions. After the images are returned to the first relative convergence measurement positions, in the processing of S9 in FIG. 4, the left eye image 200L and the right eye image 200R move from the first relative convergence measurement positions in the horizontal direction (the directions indicated by the arrows in FIG. 5(c)) of the right eye image area 111R and the left eye image area 111L, and separate from each other. The left eye image 200L and the right eye image 200R continue to be separated from each other until the predetermined operation key of the input device 115 is pressed (YES in S10 in FIG. 4). When the predetermined operation key is pressed by the measurement subject 2, the amounts of positional shift of the left eye image 200L and the right eye image 200R at this time (hereinafter referred to as "third relative convergence measurement positions" for convenience of description) are stored in the memory 114 (S11 in FIG. 4). The order of the processing of S5 or S9 and the processing of S8 in FIG. 4 can be changed as follows. As shown in FIG. 6, for example, it is possible to measure relative convergence under the conditions that the history effect of convergence is not included (SI2) by returning the left and right parallax images to the first relative convergence measurement positions (S8) before bringing or separating the parallax images closer to/from each other (S5, S9), each time the parallax images are brought closer or separated to/from each other. At this time, when two images are not viewed in the previous determination, it is preferable that the approach amount and the separation amount are respectively larger than the previous approach amount and the previous separation amount. Therefore, a step of determining the approach amount or the separation amount of parallax images before moving the parallax images may be provided (S13, S14).

In the processing of S12 in FIG. 4, a first convergence angle (divergence limit) and a second convergence angle (convergence limit) are calculated using the first relative convergence measurement positions, the second relative convergence measurement positions, the third relative convergence measurement positions, and the visual range. The first convergence angle indicates the positive relative convergence corresponding to accommodation at the visual range during measurement. The second convergence angle indicates the negative relative convergence corresponding to accommodation at the visual range during measurement. The visual range does not change during measurement. Therefore, the accommodation of the measurement subject 2 does not change substantially during measurement. Thus, the positive relative convergence and the negative relative convergence can be easily measured with high accuracy while separating the positive relative convergence and the negative relative convergence from the accommodation.

When the positive relative convergence and the negative relative convergence that are obtained in the processing of S12 in FIG. 4 are applied to the Donders diagram, the left and right Donders curves are predicted. That is, the relationship regarding cooperation between the convergence and the accommodation of the measurement subject 2 can be obtained. A Donders curve changes depending on age. Therefore, when a Donders curve is predicted, it is preferable to consider the age input as a measurement condition.

When measurement is performed in the convergence range measurement mode while changing the visual range, the positive relative convergence and the negative relative convergence obtained when different accommodation occurs are measured. As the measurement of the convergence range at different visual ranges is repeated, the number of pieces of collected sample data for predicting the Donders curves increases. Therefore, the relationship regarding cooperation between the convergence and the accommodation of measurement subject 2 can be obtained more accurately.

The operator can set and change the speed of relative changes (movement, rotation, scaling, and the like) between the left eye image 200L and the right eye image 200R as appropriate. However, it is desirable that the speed of a relative change that can be set and changed is within a predetermined range of speed. The upper limit of the speed of a relative change is set to a value such that an error caused by a time lag between the timing at which the measurement subject cannot fuse images and the timing at which the predetermined operation key of the input device 115 is pressed is within a predetermined allowable value range. On the other hand, the lower limit may be set to a value such that the display of the images changes before the fusional area expands beyond the range assumed considering the natural eyeball movement due to fusion of images being facilitated, for example. Specific examples of the set upper and lower limits are determined after performing experiments and the like, for example.

Note that, if the binocular visual function measurement program realizes the function of the tracking ability determination unit 113d, the level of the tracking ability of the eyes of the measurement subject 2 with respect to a change in the positions of the presented images may be determined by changing the speed of a change in the relative positions of the left eye image 200L and the right eye image 200R and acquiring a plurality of values in the convergence range that are predetermined parameter values. Doing this makes it possible to reflect the level of the tracking ability of the eyes of the measurement subject 2 on the moving speeds of the left eye image 200L and the right eye image 200R, and thus the measurement subject 2 can move his/her eyeballs without difficulty, and as a result, it is possible to accurately measure the binocular visual function of the measurement subject 2.

The left eye image 200L and the right eye image 200R may be separated from or brought closer to each other multiple times in order to measure the convergence range quickly and accurately. At the first measurement (hereinafter referred to as "pre-measurement" for convenience of description), for example, the left eye image 200L and the right eye image 200R are separated from or brought closer to each other at a high speed so as to specify the approximate position of the fusional limit. In the second measurement (hereinafter referred to as "main measurement" for convenience of description), for example, the left eye image 200L and the right eye image 200R are separated from or brought closer to each other at a low speed (note that, a speed at which fusion is not strong) near the approximate position specified in the pre-measurement. In the main measurement, the moving speed of the presented images is low, and thus an error caused by a time lag between the timing at which the measurement subject 2 cannot fuse images and the timing at which the predetermined operation key of the input device 115 is pressed is suppressed, and measurement accuracy is improved. Furthermore, the measurement interval in the main measurement is limited to the vicinity of the approximate position of the fusional limit specified in the pre-measurement. Thus, the convergence range can be measured quickly even if pre-measurement and main measurement are performed. Measurement items other than the convergence range are quickly measured with high accuracy, and thus pre-measurement and main measurement may be performed.

The parameter values of the convergence range of the measurement subject 2 are obtained from the positive relative convergence and the negative relative convergence that are measured in the convergence range measurement mode. The potential shift (esotropia or exotropia) of the measurement subject 2 is estimated based on such parameter values, for example. Parameter values can be estimated for measurement items other than the convergence range in a similar manner.

(If "left-right eye vertical divergence allowable value" is selected)

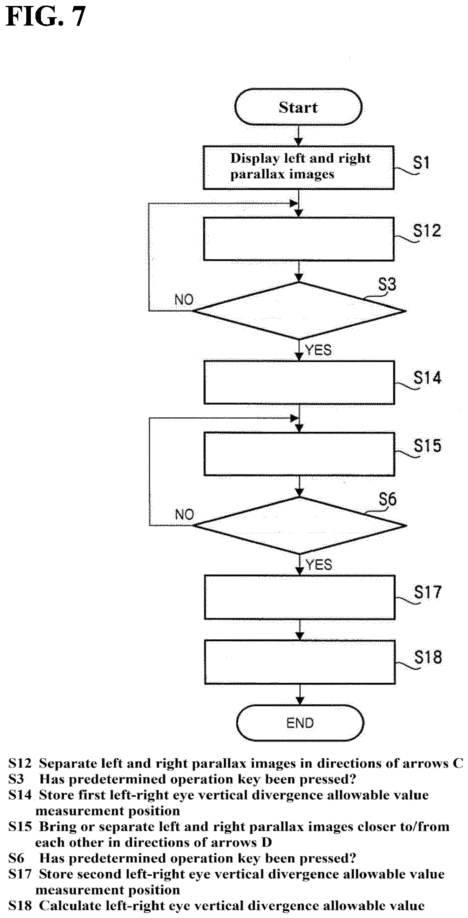

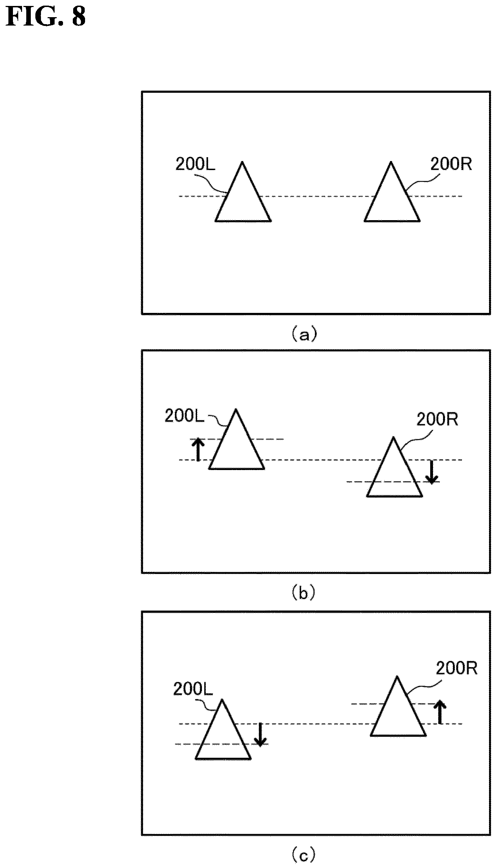

The binocular visual function measurement program transitions to the left-right eye vertical divergence allowable value measurement mode in which the left-right eye vertical divergence allowable value of the measurement subject 2 is measured. The left-right eye vertical divergence allowable value is the allowable value of vertical divergence of the left and right eyes that can enable stereoscopic vision. FIG. 7 is a diagram showing a flowchart of processing executed by the binocular visual function measurement program in the left-right eye vertical divergence allowable value measurement mode. FIG. 8 shows transition diagrams of images displayed on the display screen during execution of the left-right eye vertical divergence allowable value measurement mode. In the following description of this specification and the drawings, the same or similar processes are given the same or similar reference numerals, and description thereof will be simplified or omitted.

When the binocular visual function measurement program transitions to the left-right eye vertical divergence allowable value measurement mode and images are displayed, the left eye image 200L and the right eye image 200R are respectively displayed in the right eye image area 111R and the left eye image area 111L on the display screen 111 (SI in FIG. 7 and FIG. 8(a)) such that the left eye image 200L and the right eye image 200R are visible in a separated state. Then, as shown in FIG. 8(b), the left eye image 200L and the right eye image 200R move in the vertical direction on the display screen (the directions indicated by the arrows in FIG. 8(b)) and separate from each other (S12 in FIG. 7). When the predetermined operation key is pressed by the measurement subject 2 (YES in S3 in FIG. 7), the amounts of positional shift of the left eye image 200L and the right eye image 200R at this time (hereinafter referred to as "first left-right eye vertical divergence allowable value measurement positions" for convenience of description) are stored in the memory 114 (S14 in FIG. 7).

In the processing of S15 in FIG. 7, the left eye image 200L and the right eye image 200R move in the vertical direction on the display screen (the directions indicated by the arrows shown in FIG. 8(c) that are the opposite of the directions indicated by the arrows in FIG. 8(b)) from the first left-right eye vertical divergence allowable value measurement positions and approach each other, and separate from each other as shown in FIG. 8(c). When the predetermined operation key is pressed by the measurement subject 2 (YES in S6 in FIG. 7), the amounts of positional shift of the left eye image 200L and the right eye image 200R at this time (hereinafter referred to as "second left-right eye vertical divergence allowable value measurement positions" for convenience of description) are stored in the memory 114 (S17 in FIG. 7).

In the processing of S18 in FIG. 7, the left-right eye vertical divergence allowable value and a vertical range in which images of an object can be fused in the visual range is calculated based on the first and second left-right eye vertical divergence allowable measurement positions and the visual range. When measurement is performed in the left-right eye vertical divergence allowable value measurement mode while changing the visual range, the left-right eye vertical divergence allowable value obtained when different accommodation occurs (e.g., when a subject looks at a near position or distant position) is measured.

(If "first unequal magnification allowable value" is selected)

The first unequal magnification allowable value is the allowable value of unequal magnification of the left and right eyes that can enable stereoscopic vision. In general, whether or not to prepare a prescription of eyeglass lenses with respect to the unequal magnification is determined in a pattern-like manner in accordance with whether the eyesight difference between the left and right eyes is larger than or equal to 2 diopters. However, there are individual differences between patients, and thus, it may be difficult for a patient to achieve fusion of images even if the eyesight difference between the left and right eyes is less than 2 diopters. Also, in contrast to this, even if the eyesight difference between the left and right eyes is larger than or equal to 2 diopters, there are also cases where it may not be difficult for a patient to achieve fusion of images. In the first unequal magnification allowable value measurement mode described later, whether or not it is possible to fuse images is measured considering the eyesight difference between the left and right eyes. Thus, when the results of measurement obtained in the first unequal magnification allowable value measurement mode are used, it is possible to prepare a prescription optimal for unequal magnification with individual differences taken into consideration.

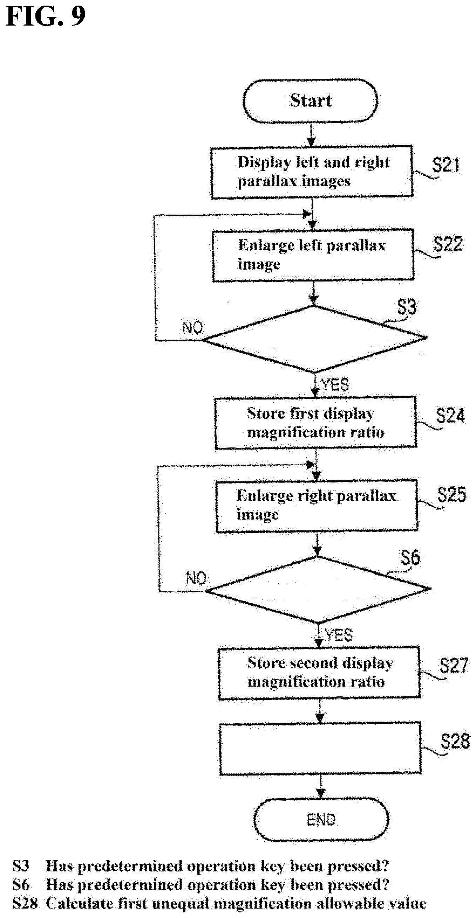

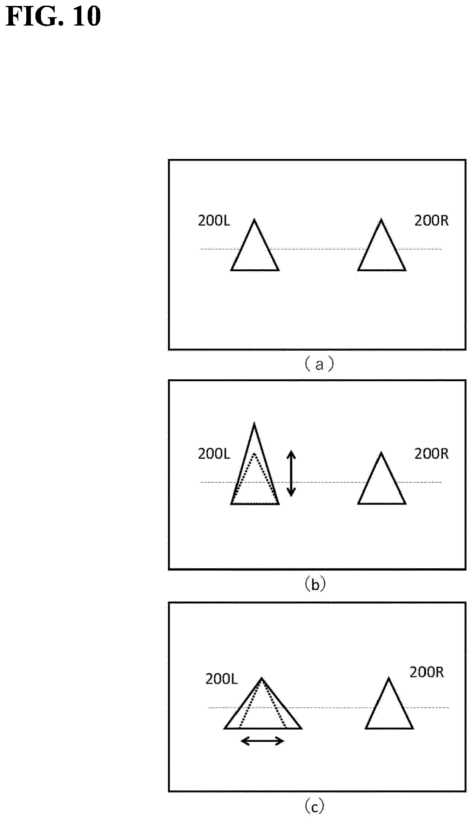

The binocular visual function measurement program transitions to the first unequal magnification allowable value measurement mode in which the first unequal magnification allowable value of the measurement subject 2 is measured. FIG. 9 is a diagram showing a flowchart of processing executed by the binocular visual function measurement program in the first unequal magnification allowable value measurement mode. FIG. 10 shows transition diagrams of images displayed on the display screen during execution of the first unequal magnification allowable value measurement mode.

When the binocular visual function measurement program transitions to the first unequal magnification allowable value measurement mode, as shown in FIG. 10(a), the left eye image 200L and the right eye image 200R are displayed at slightly shifted positions in the fusional area of the measurement subject 2 (S21 in FIG. 9). The positions where the left eye image 200L and the right eye image 200R are displayed are determined with reference to the results of measurement of the convergence range and the left-right eye vertical divergence allowable value. If the convergence range and the left-right eye vertical divergence allowable value have not been measured, the operator performs minute adjustment such that the positions of the left eye image 200L and the right eye image 200R are located in the fusional area of the measurement subject 2.

As shown in FIG. 10(b), in the processing of S22 in FIG. 9, the left eye image 200L is enlarged with respect to the right eye image 200R. The enlargement ratio of the left eye image 200L is fixed with regard to the aspect ratio, and the left eye image 200L is rendered in a continuously changing manner or an incrementally changing manner. The displayed left eye image 200L continues to be enlarged until the predetermined operation key of the input device 115 is pressed (S22 and NO in S3 in FIG. 9). When the predetermined operation key is pressed by the measurement subject 2 (YES in S3 in FIG. 9), the magnification ratio between the left eye image 200L and the right eye image 200R displayed at this time (hereinafter referred to as "first display magnification ratio" for convenience of description) is stored in the memory 114 (S24 in FIG. 9). Note that, in the first unequal magnification allowable value measurement mode, it is sufficient that the display magnification ratio between the left eye image 200L and the right eye image 200R changes relative to each other. Therefore, a change made to images may be reduction, instead of enlargement. Furthermore, during measurement, the left eye image 200L and the right eye image 200R may be enlarged or reduced simultaneously at different scaling rates. The same applies to the second unequal magnification allowable value measurement mode, which will be described later.

As shown in FIG. 10(c), in the processing of S25 in FIG. 9, the right eye image 200R is enlarged with respect to the left eye image 200L. When the predetermined operation key is pressed by the measurement subject 2 (YES in S6 in FIG. 9), the magnification ratio between the left eye image 200L and the right eye image 200R displayed at this time (hereinafter referred to as "second display magnification ratio" for convenience of description) is stored in the memory 114 (S27 in FIG. 9).

In the processing of S28 in FIG. 9, based on the first and second display magnification ratios and the visual range, the first unequal magnification allowable value in the visual range is calculated. When measurement is performed in the first unequal magnification allowable value measurement mode while changing the visual range, the first unequal magnification allowable value obtained when different accommodation occurs (e.g., when a subject looks at a near position or distant position) is measured.

(If "second unequal magnification allowable value" is selected)

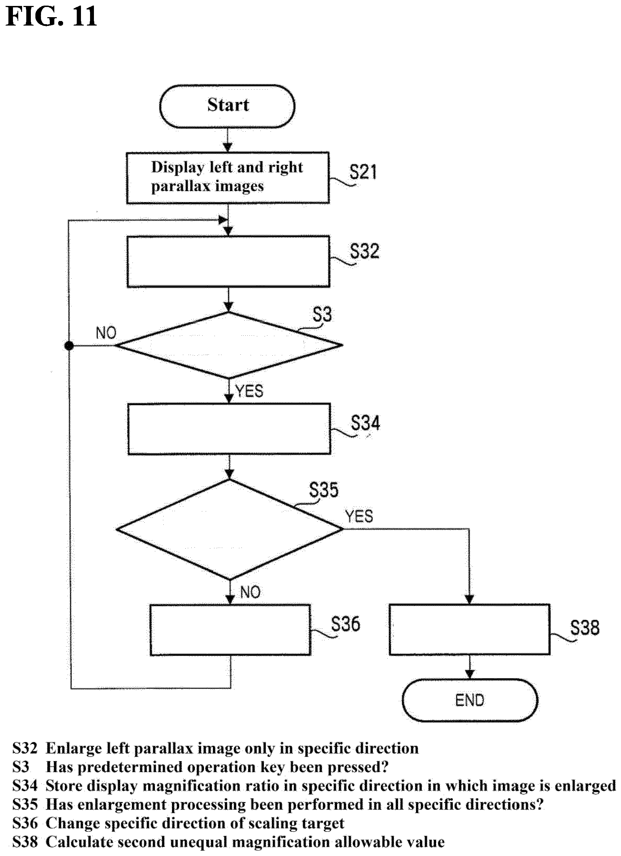

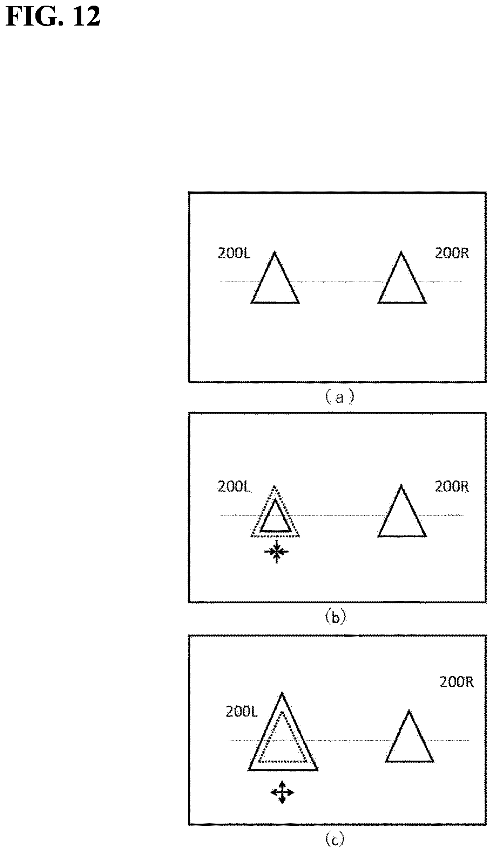

The binocular visual function measurement program transitions to the second unequal magnification allowable value measurement mode in which the second unequal magnification allowable value of the measurement subject 2 is measured. The second unequal magnification allowable value is the allowable value of unequal magnification of the left and right eyes that can enable stereoscopic vision limited to a specific direction. FIG. 11 is a diagram showing a flowchart of processing executed by the binocular visual function measurement program in the second unequal magnification allowable value measurement mode. FIG. 12 shows transition diagrams of images displayed on the display screen during execution of the second unequal magnification allowable value measurement mode.

When the binocular visual function measurement program transitions to the second unequal magnification allowable value measurement mode and images are displayed (S21 in FIG. 11, FIG. 12(a)), the left eye image 200L displayed in a specific direction is enlarged with respect to the right eye image 200R (S32 in FIG. 11). In the image example shown in FIG. 12(b), the display magnification of the left eye image 200L is increased only in the vertical direction on the screen. The enlargement of the left eye image 200L is rendered such that the size thereof changes continuously or incrementally. The displayed left eye image 200L continues to be enlarged until the predetermined operation key of the input device 115 is pressed (S32 and NO in S3 in FIG. 11). When the predetermined operation key is pressed by the measurement subject 2 (YES in S3 in FIG. 11), the display magnification ratio between the left eye image 200L and the right eye image 200R in the vertical direction on the screen at this time (hereinafter referred to as "first specific direction display magnification ratio" for convenience of description) is stored in the memory 114 (S34 in FIG. 11).

In the processing of S35 in FIG. 11, it is determined whether or not the left eye image 200L has been enlarged in all of the specific directions of scaling targets. In the present embodiment, the directions of the scaling targets are two directions including the vertical direction on the screen and the horizontal direction on the screen. Therefore, the direction of the scaling target is changed to the horizontal direction on the screen (NO in S35 and S36 in FIG. 11). Then, as shown in FIG. 12(c), the display of the left eye image 200L in the horizontal direction on the screen is enlarged with respect to the right eye image 200R. When the predetermined operation key is pressed by the measurement subject 2 (YES in S3 in FIG. 11), the display magnification ratio between the left eye image 200L and the right eye image 200R in the horizontal direction on the screen at this time (hereinafter referred to as "second specific direction display magnification ratio" for convenience of description) is stored in the memory 114 (S34 in FIG. 11).

In the processing of S38 in FIG. 11, the second unequal magnification allowable value in the visual range is calculated based on the first and second specific direction display magnification ratios and the visual range. When measurement is performed in the second unequal magnification allowable value measurement mode while changing the visual range, the second unequal magnification allowable value obtained when different accommodation occurs (e.g., when a subject looks at a near position or distant position) is measured. The directions of the scaling targets are not limited to two directions including the horizontal direction on the screen or the vertical direction on the screen, and may include other directions.

(If "left-right eye rotation parallax allowable value" is selected)

Fusional rotation may occur when the line-of-sight directions such as convergence are not parallel with each other. The rotation of an eyeball in the distance vision is based on Listing's law. Listing's law is a law defining the posture of an eyeball when the eyeball faces in a given direction in a space. The posture of the eyeball indicates the orientation of the eyeball in the lateral direction and the longitudinal direction. If the posture of the eyeball is not defined, upward, downward, left, and right directions of a retinal image are not defined. The posture of the eyeball is not defined uniquely by only the line-of-sight direction, that is, the direction of an optical axis of the eyeball. The posture of the eyeball can take all of the directions defined in regard to rotation about the line of sight serving as an axis even when the line-of-sight direction is defined.

Listing's law defines the posture of an eyeball facing an infinitely distant point in a given line-of-sight direction. With regard to Listing's law, "it is conceivable that any rotation of a single eye may occur about an axis in one plane (Listing's plane)" is described in "Handbook of Visual Information Processing" p. 405, for example.

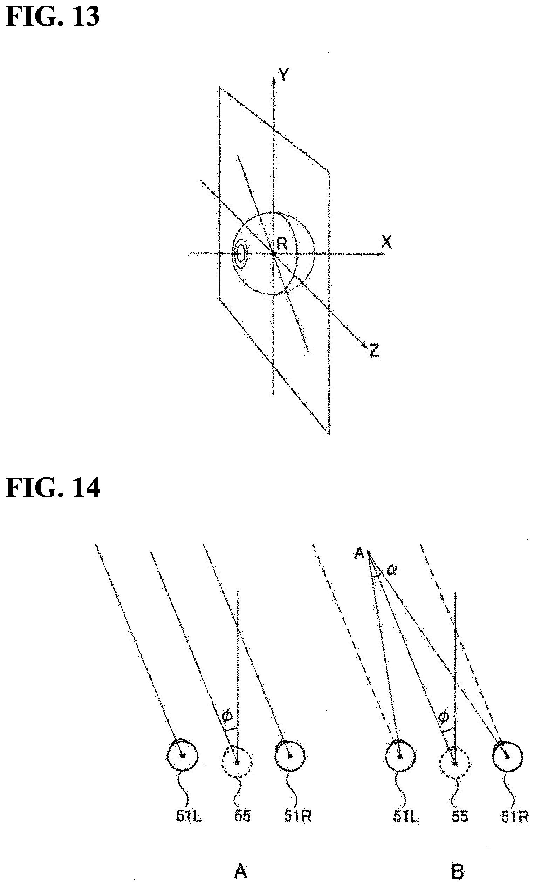

The aforementioned Listing's law will be described using a coordinate system shown in FIG. 13. The coordinate system shown in FIG. 13 is a coordinate system where a point R, which is the rotation center of an eyeball, is the origin, and a direction extending from the front plane (horizontal front side) and entering the eye is defined as the X-axial direction, a vertical direction orthogonal to the X-axial direction is defined as the Y-axial direction, and a horizontal direction orthogonal to the X-axial direction is defined as the Z-axial direction. The Y-Z plane is the Listing's plane.

The posture after rotation of an eyeball in a given direction is the same as rotation about a straight line serving as an axis in the Listing's plane including the point R. In FIG. 13, an example of straight lines serving as rotation axes are illustrated between the Y-axis and the Z-axis (on the Y-Z plane). The rotation axes are orthogonal to each of the primary eye position (X-axial direction) and the line-of-sight direction after rotation. Here, a case where an eyeball is rotated to direction vectors (L, M, N) that are not shown is considered. In this case, the vectors in the X-axial direction, the Y-axial direction, and the Z-axial direction in the eyeball coordinate system after rotation are calculated using the following equation (1).

[Mathematical 1]

$$x = L\,i + M\,j + N\,k$$

$$y = -M\,i + \left(1 - \frac{M^2}{1+L}\right)j - \frac{MN}{1+L}\,k$$

$$z = -N\,i - \frac{MN}{1+L}\,j + \left(1 - \frac{N^2}{1+L}\right)k \qquad \text{------ (1)}$$

Listing's law is appropriate in regard to a case where a single eye defines the posture of an eyeball with respect to an object at an infinite distance. Also, if a subject leans his/her body while looking at an object at an infinite distance, for example, the eyeballs of the left eye and the right eye have the same posture and the same rotation. In contrast, if a subject looks at an object that is not at an infinite distance with his/her eyes, the eyeballs of the left eye and the right eye may have different postures.

FIGS. 14A and 14B are diagrams illustrating the line-of-sight direction of the left and right eyes in the case of binocular vision. In each of FIGS. 14A and 14B, a dashed line represents a virtual eyeball 55 arranged at an intermediate position between a left eyeball 51L and a right eyeball 51R. As shown in FIG. 14A, if a subject looks at an object at an infinite distance with both eyes, the eyeball 51L and the eyeball 51R face in the same visual direction. Because the left and right eyeballs follow Listing's law, the postures thereof after rotating are also the same. At this time, there is no difference between the retinal images of the left and right eyes.

On the other hand, as shown in FIG. 14B, if a subject looks at an object (a point A) at a finite distance, convergence is required. In this case, because the visual directions of the eyeball 51L and the eyeball 51R are different from each other, the amounts of rotation of the left and right eyeballs are different from each other. In FIG. 14B, the point A is located on the forward-left side. Therefore, the amount of rotation of the eyeball 51R is larger than the amount of rotation of the eyeball 51L.

Regarding the eyeball rotation based on Listing's law, the posture of the eyeball after rotation, that is, each of the direction vectors of the Y-axis and the Z-axis after rotation, depends on the visual direction vector indicated by the equation (1). If the visual direction vectors of the left eye and the right eye are different from each other, the direction vectors of the Y-axis and the Z-axis after rotation are different between the left and right eyes. Therefore, a rotational shift occurs in the retinal images. In order to cancel the rotational shift of the retinal images, rotation about the line of sight is required for the left and right eyes. Such rotation about this line of sight is fusional rotation.

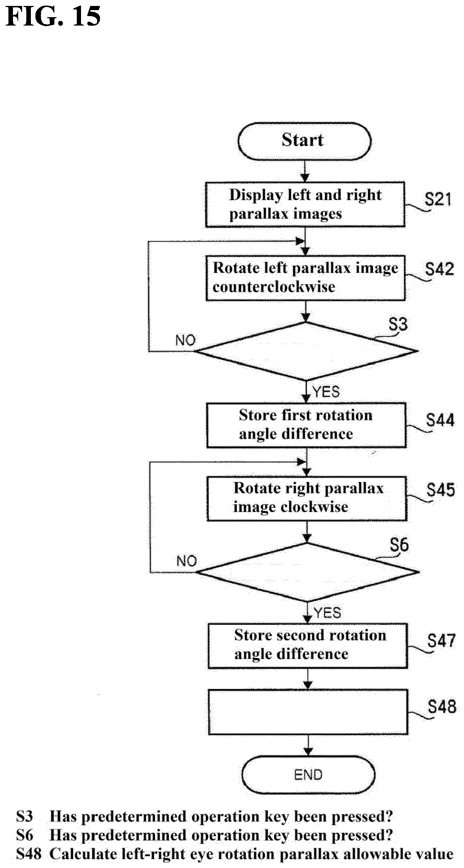

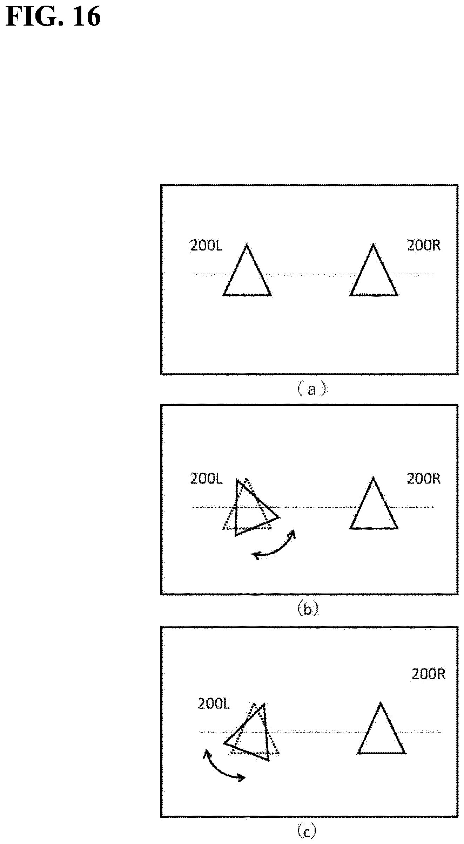

When fusional rotation occurs, rotation parallax arises between the left and right eyes. In the binocular visual function measurement program, it is possible to measure a left-right eye rotation parallax allowable value, which is an allowable value of rotation parallax of the left and right eyes that can enable stereoscopic vision. When the measurement item "left-right eye rotation parallax allowable value" is selected, the binocular visual function measurement program transitions to the left-right eye rotation parallax allowable value measurement mode in which the left-right eye rotation parallax allowable value of the measurement subject 2 is measured. FIG. 15 is a diagram showing a flowchart of processing executed by the binocular visual function measurement program in the left-right eye rotation parallax allowable value measurement mode. FIG. 16 shows transition diagrams of images displayed on the display screen during execution of the left-right eye rotation parallax allowable value measurement mode.

As shown in FIG. 16(b), when the binocular visual function measurement program transitions to the left-right eye rotation parallax allowable value measurement mode and images are displayed (S21 in FIG. 15, FIG. 16(a)), the left eye image 200L rotates counterclockwise (S42 in FIG. 15). The rotation of the left eye image 200L is rendered changing continuously or incrementally. The left eye image 200L continues to rotate until the predetermined operation key of the input device 115 is pressed (S42 and NO in S3 in FIG. 15). When the predetermined operation key is pressed by the measurement subject 2 (YES in S3 in FIG. 15), the rotation angle difference between the left eye image 200L and the right eye image 200R at this time (hereinafter referred to as a "first rotation angle difference" for convenience of description) is stored in the memory 114 (S44 in FIG. 15). Note that the center of rotation of an image is defined as the center of mass of the image. In the left-right eye rotation parallax allowable value measurement mode, it is sufficient that the rotation angle difference between the left eye image 200L and the right eye image 200R changes relative to each other. Therefore, during measurement, the left eye image 200L and the right eye image 200R may be rotated simultaneously at different speeds or in different directions.

As shown in FIG. 16(c), in the processing of S45 in FIG. 15, the left eye image 200L rotates clockwise. When the predetermined operation key is pressed by the measurement subject 2 (YES in S6 in FIG. 15), the rotation angle difference between the left eye image 200L and the right eye image 200R at this time (hereinafter referred to as a "second rotation angle difference" for convenience of description) is stored in the memory 114 (S47 in FIG. 15).

In the processing of S48 in FIG. 15, the left-right eye rotation parallax allowable value in the visual range is calculated based on the first and second rotation angle differences and the visual range. When measurement is performed in the left-right eye rotation parallax allowable value measurement mode while changing the visual range, the left-right eye rotation parallax allowable value when different accommodation occurs (e.g., when a subject looks at a near position or distant position) is measured. It is possible to optimally correct astigmatism by measuring the left-right eye rotation parallax allowable value for each of the different visual ranges and prescribing different astigmatism axes for the distance portion and the near portion, when prescribing a progressive refraction eyeglass lens, for example.

(Other measurements)

It is also possible to measure the stereoscopic function using the binocular visual function measurement system 10. In the measurement for stereoscopic vision, two types of parallax images having different shapes are displayed in the right eye image area 111R and the left eye image area 111L on the display screen 111, for example. It is desirable that a parallax image has a simple geometrical shape such as a circle or a triangle such that the measurement subject 2 can focus on measurement. In the present embodiment, the two types of parallax images are a circle image and a triangle image. The circle image has a larger degree of parallax than the triangle image has. Therefore, the measurement subject 2 sees the circle image on the near side, and sees the triangle image on the far side. Then, the parallax of at least one of the circle image and the triangle image can be changed continuously or incrementally. The measurement subject 2 presses the predetermined operation key of the input device 115, for example, when the measurement subject 2 feels that neither of the circle image and the triangle image has depth or when the measurement subject 2 sees two images. The parallax of images obtained when the predetermined operation key is pressed is stored in the memory 114. The CPU 113 calculates the limit to which the measurement subject 2 is able to perform stereoscopic viewing based on the stored parallax of images and visual range.

(Composite measurements of measurement items)

In each of the above-described various measurement modes, one measurement item is measured. In another measurement mode, a composite measurement may be performed in which composite measurement items (e.g., at least two of the convergence range, left-right eye vertical divergence allowable value, first unequal magnification allowable value, second unequal magnification allowable value, and left-right eye rotation parallax allowable value) are measured simultaneously. In particular, if a plurality of measurement items that are closely related to each other are measured simultaneously, the results of a measurement that cannot be recognized by the result of measurement of a single measurement item may be obtained. The operator can select any measurement items to be measured simultaneously. Some combinations of the measurement items may be prepared in advance. The following describes three examples of composite measurement.

(Composite measurement of convergence range - left-right eye vertical divergence allowable value)

The convergence range and the vertical divergence have strong mutual interaction, for example. In view of this, in the first composite measurement mode, the convergence range and the left-right eye vertical divergence allowable value are measured simultaneously by moving, continuously or incrementally, at least one of the left eye image 200L and the right eye image 200R in an oblique direction on the screen. Here, the "oblique direction on the screen" refers to all of the directions other than the horizontal direction on the screen or the vertical direction on the screen, and include a horizontal direction component on the screen and a vertical direction component on the screen. That is, a change of display (hereinafter referred to as a "composite change" for convenience of description) obtained by combining change patterns (movement in the horizontal direction on the screen and movement in the vertical direction of the screen) in the convergence range measurement mode and in the left-right eye vertical divergence allowable value measurement mode is given to the left eye image 200L or the right eye image 200R. The angle of the oblique direction on the screen may be set by the operator or predetermined by the binocular visual function measurement program.

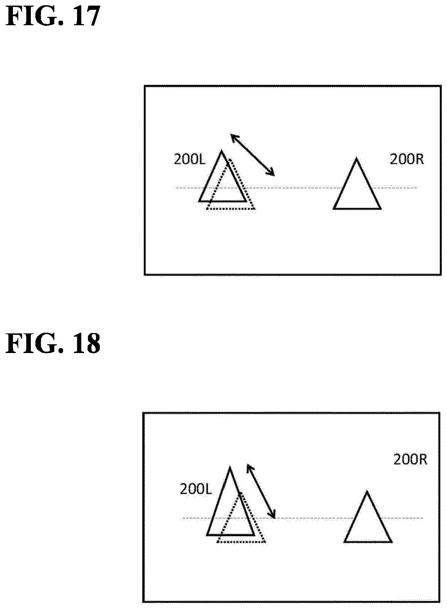

FIG. 17 shows an example of the display of the images in the first composite measurement mode. The flowchart of the first composite measurement mode is the same as the flowchart of the convergence range measurement mode and the like, and thus is omitted. According to the example in FIG. 17, the left eye image 200L moves in the oblique direction on the screen from a position indicated by the dashed line in FIG. 17. The movement in the oblique direction on the screen continues until the predetermined operation key of the input device 115 is pressed. When the predetermined operation key is pressed by the measurement subject 2, the amounts of positional shift of the left eye image 200L and the right eye image 200R at this time are stored in the memory 114. In a sequence of measurement, a plurality of pieces of data regarding the amounts of positional shift may be collected while moving the left eye image 200L or the right eye image 200R in different oblique directions on the screen. The results of composite measurement of the convergence range and the left-right eye vertical divergence allowable value in the visual range are calculated based on the amounts of positional shift and the visual range stored in the memory 114. When measurement is performed in the first composite measurement mode while changing the visual range, the results of composite measurement when different accommodation occurs (e.g., when a subject sees a near position or distant position) are obtained.

(Composite measurement of convergence range - left-right eye vertical divergence allowable value - second unequal magnification allowable value (or first unequal magnification allowable value))

The convergence range, the vertical divergence, and unequal magnification of the left and right eyes have strong mutual interaction, for example. In view of this, in the second composite measurement mode, at least one of the left eye image 200L and the right eye image 200R is moved in the oblique direction on the screen continuously or incrementally, and is displayed in an enlarged or reduced size. If enlargement or reduction of an image is limited to a specific direction, the second unequal magnification allowable value is measured, whereas, if an image is enlarged or reduced in a fixed aspect ratio, the first unequal magnification allowable value is measured. A composite change, which is obtained by combining the changing patterns (movement in the horizontal direction on the screen, movement in the vertical direction on the screen, a change in display magnification) in the convergence range measurement mode, left-right eye vertical divergence allowable value measurement mode, or second unequal magnification allowable value (or first unequal magnification allowable value) measurement mode, is given to the left eye image 200L or the right eye image 200R. The ratio at which each change pattern is given may be set by the operator or may be predetermined by the binocular visual function measurement program.
FIG. 18 shows an example of the display of the images in the second composite measurement mode. The flowchart of the second composite measurement mode is the same as the flowchart of the convergence range measurement mode and the like, and thus is omitted. According to the example in FIG. 18, the left eye image 200L moves in the oblique direction on the screen from a position indicated by the dashed line in FIG. 18, and is enlarged only in the vertical direction on the screen. The left eye image 200L continues to be moved and enlarged until the predetermined operation key of the input device 115 is pressed. When the predetermined operation key is pressed by the measurement subject 2, the amounts of positional shift of the left eye image 200L and the right eye image 200R and the display magnification ratio in the vertical direction on the screen (hereinafter referred to as an "image change state" for convenience of description) at this time are stored in the memory 114. In a sequence of measurement, a plurality of pieces of data regarding image change states may be collected while repeating movement and enlargement of the left eye image 200L or the right eye image 200R in different patterns. The results of composite measurement of the convergence range, the left-right eye vertical divergence allowable value, and the second unequal magnification allowable value (or the first unequal magnification allowable value) in the visual range are calculated based on the image change states and the visual range stored in the memory 114. When measurement is performed in the second composite measurement mode while changing the visual range, the results of composite measurement when different accommodation occurs (e.g., when a subject looks at a near position or distant position) are obtained.

(Composite measurement of convergence range - left-right eye rotation parallax allowable value)

As described above, fusional rotation occurs accompanying convergence. In view of this, in the third composite measurement mode, at least one of the left eye image 200L and the right eye image 200R is moved in the horizontal direction on the screen continuously or incrementally, and is rotated clockwise or counterclockwise. That is, a composite change obtained by combining change patterns (movement in the horizontal direction on the screen and rotation about the center of mass of the image) in the convergence range measurement mode and in the left-right eye rotation parallax allowable value measurement mode is given to the left eye image 200L or the right eye image 200R. The ratio at which each change pattern is given may be set by the operator or may be predetermined by the binocular visual function measurement program.

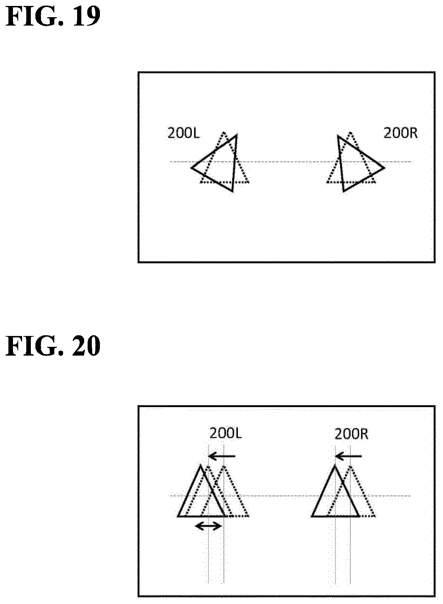

FIG. 19 shows an example of the display of the images in the third composite measurement mode. The flowchart of the third composite measurement mode is the same as the flowchart of the convergence range measurement mode or the like, and thus is omitted. According to the example in FIG. 19, the left eye image 200L and the right eye image 200R move in the horizontal direction on the screen and rotate counterclockwise and clockwise while separating from each other. The left eye image 200L and the right eye image 200R continue to be moved and rotated until the predetermined operation key of the input device 115 is pressed. When the predetermined operation key is pressed by the measurement subject 2, the amounts of positional shift of the left eye image 200L and the right eye image 200R and the rotation angle difference therebetween at this time are stored in the memory 114. In a sequence of measurement, a plurality of pieces of data regarding the amounts of positional shift and the rotation angle difference may be collected while repeating movement and rotation of the left eye image 200L or the right eye image 200R in different patterns. The results of composite measurement of the convergence range and the left-right eye rotation parallax allowable value in the visual range are calculated based on the amounts of positional shift, the rotation angle difference, and the visual range stored in the memory 114. When measurement is performed in the third composite measurement mode while changing the visual range, the results of composite measurement when different accommodation occurs (e.g., when a subject looks at a near position or distant position) are obtained.

(Measurement in consideration of lateral view)

In each of the above-described measurement modes, the left eye image 200L and the right eye image 200R are displayed in the center portion of the display screen. Therefore, this measurement only provides the results of measurement performed in a state where the measurement subject 2 faces forward. In view of this, after measurement in the state where the measurement subject 2 faces forward in each measurement mode is complete, as shown in FIG. 20, the positions where the left eye image 200L and the right eye image 200R are displayed are moved to a peripheral portion (the upper left corner of the screen) of the display screen, for example. Because the support housing portion 112a supporting the smartphone 110 is worn on the head of the measurement subject 2 and the positional relationship therebetween is fixed, the left eye image 200L and the right eye image 200R are viewed from lateral sides. When measurement is performed in this state, the results of measurement in a state where the measurement subject 2 looks at the images from a lateral side are obtained. Furthermore, when measurement is performed while successively moving the left eye image 200L and the right eye image 200R to different positions in the peripheral portion on the screen (e.g., the center portion of the upper end of the screen, the upper right corner of the screen, the center portion of the right end of the screen, the lower right corner of the screen,...), the results of measurement in the lateral view state in various directions are obtained. A lateral view differs from a front view in conditions such as fusional rotation always being involved, for example. Therefore, results of measurement that are different from those in a front view are obtained. If an eyeglass lens is designed in consideration of such results of measurement, a more suitable prescription can be obtained.

<Effects of the Present Embodiment>

According to the present embodiment, one or more effects described below can be obtained.

(a) In the present embodiment, when the binocular visual function of the measurement subject 2 is measured, a right eye image and a left eye image (i.e., parallax images) are presented to the measurement subject 2 on a single portable display screen 111. Specifically, the display screen 111 of the smartphone 110 is divided into the right eye image area 111R and the left eye image area 111L, and the parallax images are presented to the measurement subject 2 by displaying the right eye image in the right eye image area 111R and the left eye image in the left eye image area 111L, for example. Therefore, it is possible to present the parallax images using a very simple configuration such as the single display screen 111 without requiring a large-scale system configuration such as a stationary three-dimensional compatible video monitor, which is very preferable in order to simplify measurement of the binocular visual function of the measurement subject 2.

Also, by using the portable display screen 111, the display screen 111 can be very easily positioned in front of the eyes of the measurement subject 2. It is very preferable to use this display screen 111 in order to simplify measurement of the binocular visual function of the measurement subject 2. Furthermore, by keeping the display screen 111 positioned in front of the eyes of the measurement subject 2, an error in the positions of the left and right parallax images with respect to the median plane can be kept constant regardless of the direction of the face of the measurement subject 2. Therefore, it is preferable to attach the display screen 111 to the measurement subject 2 such that no error occurs in the positions of the left and right parallax images.

Furthermore, if the portable display screen 111 is used, it is possible to easily present parallax images in a space in which real space information is blocked out by screening out the surrounding portion thereof. If the parallax images are presented in a space in which real space information is blocked out, the measurement subject 2 does not acquire information (real space information) that gives a sense of depth and perspective from the outside world, in addition to the presented parallax images. Therefore, it is possible to precisely measure the capability relating to the binocular visual function without the measurement subject 2 relying on the sense of depth.

That is, according to the present embodiment, the binocular visual function of the measurement subject 2 can be very easily measured with high accuracy by presenting the parallax images on the single portable display screen 111.

(b) In the present embodiment, the binocular visual function of a measurement subject is measured using the display screen 111 of the smartphone 110, which is a mobile information terminal of one type, as a portable display screen. Thus, it is possible to easily and reliably present parallax images on a single portable display screen, thus suppressing installation costs therefore. Therefore, the realization of the presentation of parallax images thereon is preferable in order to very easily measure the binocular visual function with high accuracy.

(c) As described in the present embodiment, if values for specifying the convergence range of the measurement subject 2 are calculated as predetermined parameter values for the binocular visual function of the measurement subject 2, it is possible to very easily measure the convergence range of the measurement subject 2 with high accuracy. Also, by using the results of the above measurement as one of the parameters for designing an eyeglass lens, it is possible to provide an eyeglass lens suitable for the measurement subject 2.

(d) As described in the present embodiment, if the level of the ability of an eye of the measurement subject 2 to track a change in positions of the presented images is determined and the speed of a change in the positions of the presented images is then determined based on the results of the determination, the level of the tracking ability of the eye of the measurement subject 2 reflects on the speed of a change in the position of an image presented on the left side, and thus the measurement subject 2 can move his/her eyeballs without difficulty. As a result, it is possible to measure the binocular visual function of the measurement subject 2 with high accuracy.

<Variations and the like>

Although the embodiment of the present invention was described above, the disclosed content described above illustrates exemplary embodiments of the present invention. That is to say, the technical scope of the present invention is not limited to the above-described exemplary aspects, and various modifications can be made without departing from the gist thereof.

Although the case where the parallax images are presented on a single portable display screen using the display screen 111 of the smartphone 110 was described as an example in the above-described embodiment, the present invention is not limited to this, and a configuration may be adopted in which parallax images are presented using the display screen of another mobile information terminal such as a tablet terminal or PDA, for example.

Also, the above-described embodiment was described on the premise that the moving speed, rotation speed, and scaling speed of the left eye image 200L or the right eye image 200R are kept constant, for example. However, the present invention is not limited to this, and movement, rotation, or scaling of the left eye image 200L or the right eye image 200R may be accelerated.

List of Reference Numerals

| | |
|---|---|
| 1 | Eyeglass lens manufacturing system |
| 10 | Binocular visual function measurement system |
| 20 | Input device |
| 30, 130 | PC |
| 40, 140 | Display |
| 50 | Processing device |
| 110 | Smartphone |
| 111 | Display screen |
| 111R | Right eye image area |
| 111L | Left eye image area |
| 113 | CPU |
| 113a | Presentation control unit |
| 113b | Timing detection unit |
| 113c | Parameter value calculation unit |
| 113d | Tracking ability determination unit |
| 200R | Right eye image |
| 200L | Left eye image |

**Claims**

1. A binocular visual function measurement method comprising:

   a visual target presentation step of presenting a right eye image to be viewed by the right eye of a measurement subject and a left eye image to be viewed by the left eye of the measurement subject to the measurement subject on a single portable display screen;
   a presentation control step of changing positions where the right eye image and the left eye image are presented, relative to each other;
   a timing detection step of detecting a timing at which the measurement subject is unable to fuse the right eye image and the left eye image when the presentation positions are changed; and
   a parameter value calculation step of calculating a predetermined parameter value regarding a binocular visual function of the measurement subject based on a relationship between the relative positions of the right eye image and the left eye image when the timing is detected.

2. The binocular visual function measurement method according to claim 1,
   wherein the right eye image and the left eye image are presented using a display screen of a mobile information terminal as the portable display screen.

3. The binocular visual function measurement method according to claim 1 or 2,
   wherein the predetermined parameter value is a value for specifying a convergence range of the measurement subject.

4. The binocular visual function measurement method according to any one of claims 1 to 3, further comprising a tracking ability determination step of determining a level of the ability of an eye of the measurement subject to track a change in positions of the presented images by changing the speed of a change in the relative positions of the right eye image and the left eye image and acquiring a plurality of the predetermined parameter values.

5. The binocular visual function measurement method according to any one of claims 1 to 4,
   wherein the right eye image and the left eye image are constituted by figures having the same shape and the same size.

6. The binocular visual function measurement method according to any one of claims 1 to 5, further comprising a visual range setting step of setting a visual range of the measurement subject with respect to the right eye image and the left eye image.

7. A binocular visual function measurement program for causing a computer to execute the binocular visual function measurement method according to any one of claims 1 to 6.

8. An eyeglass lens designing method comprising:

   a step of measuring the binocular visual function of the measurement subject using the binocular visual function measurement method according to any one of claims 1 to 6; and
   a step of determining an optical design value of the eyeglass lens based on a result of the measurement of the binocular visual function.

9. An eyeglass lens manufacturing method comprising:

   a step of designing an eyeglass lens using the eyeglass lens designing method according to claim 8; and
   a step of manufacturing the eyeglass lens according to a result of designing the eyeglass lens.

10. A binocular visual function measurement system comprising:

    a visual target presentation unit configured to present a right eye image to be viewed by the right eye of a measurement subject and a left eye image to be viewed by the left eye of the measurement subject to the measurement subject on a single portable display screen;
    a presentation control unit configured to change positions where the right eye image and the left eye image are presented, relative to each other;
    a timing detection unit configured to detect a timing at which the measurement subject is unable to fuse the right eye image and the left eye image when the presentation positions are changed; and
    a parameter value calculation unit configured to calculate a predetermined parameter value regarding a binocular visual function of the measurement subject based on a relationship between the relative positions of the right eye image and the left eye image when the timing is detected.

11. The binocular visual function measurement system according to claim 10,
    wherein the visual target presentation unit is configured to present the right eye image and the left eye image using a display screen of a mobile information terminal as the portable display screen.

# FIG. 1

10 = Binocular visual function
measurement system

# FIG. 2

# FIG. 3

(a)

111    110

Left eye image area    Right eye image area

111L    111R

(b)

110

**Smartphone**

113

CPU

113a

113b

113c

113d

Memory    114

Input device    115

Display screen    111

130    PC

113a  Presentation control unit    113c  Parameter value calculation unit
113b  Timing detection unit    113d  Tracking ability determination unit

# FIG. 4

S1 Display left and right parallax images
S2 Has predetermined key been pressed? (two images are seen)
S3 Bring or separate left and right parallax images
    closer to/from each other
S4 Store first relative convergence measurement positions
S5 Bring left and right parallax images closer to each other
S6 Has predetermined key been pressed? (two images are seen)

S7 Store second relative convergence measurement positions
S8 Return parallax images to first relative
    convergence measurement positions
S9 Separate left and right parallax images from each other
S10 Has predetermined key been pressed?
    (two images are seen)
S11 Store third relative convergence measurement positions
S12 Calculate positive relative convergence and
    negative relative convergence

# FIG. 5

(a)

(b)

(c)

# FIG. 6

S1 Display left and right parallax images
S2 Has predetermined key been pressed?
   (two images are seen)
S3 Bring or separate left and right parallax
   images closer to/from each other
S4 Store first relative convergence measurement positions
S8 Return parallax images to first relative
   convergence measurement positions
S13 Determine approach amounts of parallax images
S5 Bring left and right parallax images
   closer to each other
S6 Has predetermined key been pressed?
   (two images are seen)

S7 Store second relative convergence
   measurement positions
S8 Return parallax images to first relative
   convergence measurement positions
S14 Determine separation amount of parallax images
S9 Separate left and right parallax images
   from each other
S10 Has predetermined key been pressed?
   (two images are seen)
S11 Store third relative convergence
   measurement positions
S12 Calculate positive relative convergence
   and negative relative convergence

# FIG. 7

```
        ┌──────────┐
        │  Start   │
        └────┬─────┘
             ▼
    ┌──────────────────┐
    │ Display left and │  S1
    │ right parallax   │
    │ images           │
    └────────┬─────────┘
             ▼
    ┌──────────────────┐
    │                  │  S12
    └────────┬─────────┘
             ▼
  NO      ◇◇◇◇◇◇◇        S3
  ◀───────◇       ◇
          ◇◇◇◇◇◇◇
             │ YES
             ▼
    ┌──────────────────┐
    │                  │  S14
    └────────┬─────────┘
             ▼
    ┌──────────────────┐
    │                  │  S15
    └────────┬─────────┘
             ▼
  NO      ◇◇◇◇◇◇◇        S6
  ◀───────◇       ◇
          ◇◇◇◇◇◇◇
             │ YES
             ▼
    ┌──────────────────┐
    │                  │  S17
    └────────┬─────────┘
             ▼
    ┌──────────────────┐
    │                  │  S18
    └────────┬─────────┘
             ▼
        ┌──────────┐
        │   END    │
        └──────────┘
```

S12  Separate left and right parallax images in directions of arrows C
S3   Has predetermined operation key been pressed?
S14  Store first left-right eye vertical divergence allowable value
       measurement position
S15  Bring or separate left and right parallax images closer to/from
       each other in directions of arrows D
S6   Has predetermined operation key been pressed?
S17  Store second left-right eye vertical divergence allowable value
       measurement position
S18  Calculate left-right eye vertical divergence allowable value

# FIG. 8

(a)

(b)

(c)

# FIG. 9

Start

Display left and right parallax images — S21

Enlarge left parallax image — S22

S3

NO

YES

Store first display magnification ratio — S24

Enlarge right parallax image — S25

S6

NO

YES

Store second display magnification ratio — S27

S28

END

S3  Has predetermined operation key been pressed?
S6  Has predetermined operation key been pressed?
S28 Calculate first unequal magnification allowable value

# FIG. 10

(a)

(b)

(c)

# FIG. 11

```
                    ┌─────────────┐
                    │    Start    │
                    └──────┬──────┘
                           │
                    ┌──────▼──────────┐
                    │ Display left and│  S21
                    │ right parallax  │
                    │ images          │
                    └──────┬──────────┘
       ┌───────────────────│
       │            ┌──────▼──────────┐
       │            │                 │  S32
       │            │                 │
       │            └──────┬──────────┘
       │                   │
       │      NO    ╱──────▼──────╲   S3
       ●───────────╱               ╲
                   ╲               ╱
                    ╲─────┬───────╱
                          │YES
                    ┌─────▼───────┐
                    │             │  S34
                    │             │
                    └─────┬───────┘
                          │
                    ╱─────▼───────╲  S35
                   ╱               ╲     YES
                   ╲               ╱──────────────┐
                    ╲─────┬───────╱               │
                          │NO                     │
                    ┌─────▼───────┐        ┌──────▼──────┐
                    │             │  S36   │             │  S38
                    │             │        │             │
                    └─────────────┘        └──────┬──────┘
                                                  │
                                           ┌──────▼──────┐
                                           │     END     │
                                           └─────────────┘
```

S32 Enlarge left parallax image only in specific direction
S3  Has predetermined operation key been pressed?
S34 Store display magnification ratio in specific direction in which image is enlarged
S35 Has enlargement processing been performed in all specific directions?
S36 Change specific direction of scaling target
S38 Calculate second unequal magnification allowable value

# FIG. 12

(a)

(b)

(c)

## FIG. 13

## FIG. 14

A

B

# FIG. 15

```
            ┌─────────────┐
            │    Start     │
            └──────┬──────┘
                   │
            ┌──────▼──────────┐
            │ Display left and right │  S21
            │  parallax images │
            └──────┬──────────┘
                   │
            ┌──────▼──────────┐
   ┌───────►│ Rotate left parallax image │  S42
   │        │  counterclockwise │
   │        └──────┬──────────┘
   │               │
   │          ◇────▼────◇  S3
   │    NO   ◇          ◇
   └─────────◇          ◇
              ◇────┬────◇
                   │ YES
            ┌──────▼──────────┐
            │ Store first rotation │  S44
            │  angle difference │
            └──────┬──────────┘
                   │
            ┌──────▼──────────┐
   ┌───────►│ Rotate right parallax │  S45
   │        │  image clockwise │
   │        └──────┬──────────┘
   │               │
   │          ◇────▼────◇  S6
   │    NO   ◇          ◇
   └─────────◇          ◇
              ◇────┬────◇
                   │ YES
            ┌──────▼──────────┐
            │ Store second rotation │  S47
            │  angle difference │
            └──────┬──────────┘
                   │
            ┌──────▼──────────┐
            │                  │  S48
            └──────┬──────────┘
                   │
            ┌──────▼──────┐
            │    END       │
            └─────────────┘
```

S3 Has predetermined operation key been pressed?
S6 Has predetermined operation key been pressed?
S48 Calculate left-right eye rotation parallax allowable value

# FIG. 16

(a)

(b)

(c)

**FIG. 17**

**FIG. 18**

**FIG. 19**

**FIG. 20**

## INTERNATIONAL SEARCH REPORT

| | International application No. |
| --- | --- |
| | PCT/JP2020/031783 |

**A. CLASSIFICATION OF SUBJECT MATTER**
Int.Cl. A61B3/08(2006.01)i, G02B30/00(2020.01)i, G02C7/02(2006.01)i
FI: A61B3/08, G02C7/02, G02B30/00

According to International Patent Classification (IPC) or to both national classification and IPC

**B. FIELDS SEARCHED**

Minimum documentation searched (classification system followed by classification symbols)
Int.Cl. A61B3/00-3/18, G02B30/00-30/60, G02C1/00-13/00

Documentation searched other than minimum documentation to the extent that such documents are included in the fields searched
    Published examined utility model applications of Japan      1922-1996
    Published unexamined utility model applications of Japan    1971-2020
    Registered utility model specifications of Japan            1996-2020
    Published registered utility model applications of Japan    1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2012-95693 A (HOYA CORPORATION) 24.05.2012 (2012-05-24), paragraphs [0031]-[0092], fig. 1-14 | 1-11 |
| A | JP 2008-86657 A (HANDA, Tomoya) 17.04.2008 (2008-04-17), paragraphs [0040]-[0071], fig. 1-11 | 1-11 |
| A | JP 2015-524943 A (ESSILOR INTERNATIONAL (COMPAGNIE GENERALE D'OPTIQUE)) 27.08.2015 (2015-08-27), paragraphs [0026]-[0082], fig. 1-3 | 1-11 |

☐ Further documents are listed in the continuation of Box C.  ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 25.09.2020 | 13.10.2020 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

INTERNATIONAL SEARCH REPORT

International application No.

Information on patent family members

PCT/JP2020/031783

| | | |
|---|---|---|
| JP 2012-95693 A   24.05.2012 | US 2012/0105609 A1 paragraphs [0049]-[0111], fig. 1-14 DE 102011054833 A1 | |
| JP 2008-86657 A   17.04.2008 | (Family: none) | |
| JP 2015-524943 A  27.08.2015 | US 2014/0028972 A1 paragraphs [0048]-[0114], fig. 1-3 WO 2014/019969 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012095693 A

- WO 2010090144 A

**Non-patent literature cited in the description**

- **SHINICHI SHIKANO.** Little Pupil Science. Kanehara & Co. Ltd, 1925, 50

- **TOYOHIKO HATADA.** Depth Information and Characteristics of Vision. Visual Information Research Group, 23 April 1974, 12